# EUROPEAN PATENT SPECIFICATION

(11) **EP 2 472 440 B1**
(45) Date of publication and mention of the grant of the patent: **25.06.2014**
(21) Application number: 11150047.6
(22) Date of filing: 04.01.2011
(51) Int. Cl.: G06N 3/04, G06N 3/08

(54) **Method and system for diagnosis of plant status**
Verfahren und System zur Diagnose des Zustands einer Anlage
Procédé et système pour le diagnostic du statut d'une installation

(43) Date of publication of application: 04.07.2012
(73) Proprietor: Siemens Aktiengesellschaft, 80333 München (DE)
(72) Inventor: Bierweiler, Thomas, 76297, Stutensee (DE); Kirchberg, Karl-Heinz, 76149, Karlsruhe (DE); Lenz, Henning, 76135, Karlsruhe (DE)

(56) References cited:
- EP-A1- 1 640 894
- EP-A1- 1 777 601
- S. -L. Jamsa-Jounela, M. Vermasvuori, P. Enden, S. Haavisto: "A process monitoring system based on the Kohonen self-organizing maps", ScienceDirect Control Engineering Practice, vol. 11, no. 1 January 2003 (2003-01), pages 83-92, XP002636558, ISSN: 0967-0661, DOI: 10.1016/S0967-0661(02)00141-7 Retrieved from the Internet: URL:http://www.sciencedirect.com/science/a rticle/B6V2H-479K2T9-4/2/e54e1140766e730cd 14c96247dc736dd [retrieved on 2011-05-12]
- VACHKOV G ET AL: "Real-Time Classification Algorithm for Recognition of Machine Operating Modes by Use of Self-Organizing Maps", ELEKTRIK, SCIENTIFIC AND TECHNICAL RESEARCH COUNCIL OF TURKEY, ANKARA, TR, vol. 12, no. 1, 1 March 2004 (2004-03-01), pages 27-42, XP002570780, ISSN: 1300-0632
- L.M. BARTLETTE.E. HURDLEE.M. KELLY: "Integrated System Fault Diagnostics Utilising Digraph and Fault Tree Based Approaches", RELIABILITY ENGINEERING AND SYSTEM SAFETY, vol. 94, no. 6, 20 September 2001 (2001-09-20), pages 1107-1115, XP026001507,
- Geert Craessaerts and Josse De Baerdemaeker and Wouter Saeys: "Fault diagnostic systems for agricultural machinery", ScienceDirect Biosystems Engineering, vol. 106, no. 1 1 May 2010 (2010-05-01), pages 26-36, XP002636559, ISSN: 1537-5110, DOI: 10.1016/j.biosystemseng.2009.12.004 Retrieved from the Internet: URL:http://www.sciencedirect.com/science/a rticle/B6WXV-4YRGJF3-1/2/ad15b95012d2933aa 1f0e904df153fe2 [retrieved on 2011-05-21]
- R. A. Vingerhoeds, P. Janssens, B. D. Netten, M. Aznar Fernandez-Montesinos: "Enhancing off-line and on-line condition monitoring and fault diagnosis", ScienceDirect Control Engineering Practice, vol. 3, no. 11 November 1995 (1995-11), pages 1515-1528, XP002636560, ISSN: 0967-0661, DOI: 10.1016/0967-0661(95)00162-N Retrieved from the Internet: URL:http://www.sciencedirect.com/science/a rticle/B6V2H-3YK00G9-H/2/3d62b9f1a2fd6b418 bee949b9f0f0615 [retrieved on 2011-05-12]

## Description

The present invention relates to diagnosis of plant status, and in particular, to a method and system for diagnosing plant status by deploying self-organizing maps.

Fault detection and diagnosis is an important problem in process engineering. Early detection and diagnosis of process faults while the plant is still operating in a controllable region can help avoid abnormal event progression and reduce productivity loss.

Today's plants in process automation, for example, in the fields of chemical, pharmaceutical, food and beverage, oil and gas, among others, are often very complex and sometimes feature several thousand transmitters. The transmitters themselves increasingly feature good self-diagnosis capabilities. Nevertheless, there are good reasons for a comprehensive plant or unit diagnosis. These reasons comprise faults due to control problems, failures of non-intelligent devices or parts, faulty operation by plant personnel. What is more, self-diagnosis features of transmitters may fail, and thus an additional, independent diagnosis is desirable.

Research is ongoing on several methods concerning fault detection and diagnosis of plants. Such method include, for example, process history based methods, which usually rely on a large data base of process data with known failure state. An example of such a method includes using self-organizing maps (SOM), also known as Kohonen-maps. The objective of these maps is to automatically group similar states together. The performance of the map depends on several circumstances, among which is the size of the map, the learning rate and learning function, the neighborhood function, the number of training phases, the amount of input data, the weighting of the input data, the quality of the input data and the initial state of the map. Usually, data is learned unsupervised. The final map may be altered by supervised learning.

Broadly speaking, a SOM may be trained based on two approaches. In a first approach, the SOM is learned using historical process data with state known to be good. During operation, process data is fed into the map. The distance of the input data vector to the best matching neuron in the trained map is calculated. If the distance is greater than a certain threshold, a failure state or unknown state is assumed. The second approach, the SOM is learned using historical process data with known state. The position of the data is being tracked. Nodes which contain bad states are being marked. Accordingly, nodes which contain good states are marked. During operation, process data is fed into the map. If data is mapped to a node that has been marked as "good", a good state is assumed. Accordingly, if data is mapped to a node that has been marked as "bad", a bad state is assumed. What is more, if the distance of the process data from the map is greater than a certain threshold, an unknown state is assumed.

Both approaches for training a map have the disadvantage that many different process states may be valid process states. This makes it quite difficult to correctly differentiate between good and bad states. To be more specific, certain values of a data set may indicate a good condition within a first process state, whereas they may indicate a bad condition within a second process state. Thus, if in the second process state this kind of bad condition occurs, it will (incorrectly) be indicated as a good state in the map.

The document Dr. Gregor Schmitt-Pauksztat, Dr. Stefan Ochs, Dr. Reiner Hotop, Christian W. Frey, Dr. Helge-Bjorn Kuntze; Process Unit Monitoring mit Verfahren des maschinellen Lernens, VDI-Berichte Nr. 2032, 2008, p. 219ff describes a method of online-monitoring parts of plants and processes. Historical process data of which the state is known to be good is used to set-up and train a self-organizing map. Fault conditions can be detected as deviation from normal state. Feasible sizes of the map, however, are not discussed in the above article.

In the document WO 03063017 A2**,** a method is disclosed which targets the learning rate of self-organizing maps. Non-monotonical learning functions are introduced. This increases the rate of convergence and thus leads to a faster calculation of the map.

The document EP 2003604 A1 discloses a method on how to map information items such that similar information items are mapped to similar positions. This results in a reduced storage capacity and the time needed for determining the matching position for the information item.

The document WO 2009/126815 A2 discloses a method that involves maintaining a self-organizing map (SOM) database containing user preference data entries that include wide range of fields or attributes of user preferences. A set of SOMs is created by filtering from the SOM database. A set of trained SOMs is applied in coordination with each other, where the trained SOMs provide recommendations in response to the queries based upon inter-cooperation of the SOMs trained with respective fields and attributes of user preferences related to respective SOMs.

The document S. -L. Jamsa-Jounela, M. Vermasvuori, P. Enden, S. Haavisto: "A process monitoring system based on the Kohonen self-organizing maps", ScienceDirect, Control Engineering Practice, vol. 11, no. 1 January 2003 (2003-01), pages 83-92, XP002636558, ISSN: 0967-0661, DOI: 10.1016/S0967-0661(02)00141-7, describes a method for diagnosis of status of a process plant, comprising: obtaining historical process data of said plant, grouping said historical process data into a plurality of time based groups and a plurality of spatial groups, training a plurality of self-organizing maps using said historical process data, wherein each self-organizing map corresponds to a combination of said time based groups and said spatial groups, deploying the trained self-organizing maps under plant operation, comprising feeding the trained self-organizing maps with current process data of said plant, and determining a fault condition when a deployed self-organizing map detects a deviation in the current process data; the spatial grouping is done by extracting engineering data of the plant, the engineering data comprising data containing information about logical connections of parts of the plant and information about the signals from different devices in the plant, said spatial grouping comprises using that engineering data to define separating parts in the plant.

The object of the present invention is to reduce the size of self-organizing maps deployed for diagnosis of plant status, while providing an improved accuracy in differentiation between good and bad states.

The above object is achieved by the method according to claims 1 and the system according to claim 13.

The underlying idea of the present invention is that historical process data is evaluated by a self-organizing map wherein such historical process data is grouped in at least one of two ways, namely, time based grouping and spatial grouping. A separate self-organizing map is trained for each spatial and/or time based group. These self-organizing maps are then deployed to detect faults using current process data. The above-mentioned time based and/or spatial grouping provides several advantages. First, the map size of each self-organizing map can be smaller compared to using a single map. Searching the node with the smallest distance during operation can then be performed faster. More importantly, the number of different good states in each map is much smaller than using a single map. Thus, outliers can be identified much easier and more accurate. As described by means of an example provided below, using a small map size is sufficient to detect various types of faults.

Several embodiments may be considered while practicing the invention. For example, for continuous processes, using time based groups can be omitted. Again, if splitting the plant into spatial groups is not possible or does not make sense, e.g. for very small plants, using spatial groups can be omitted. However, as seen in the description below, the best results are achieved when both time based and spatial grouping is carried out. It has been shown by way of the description below that the use of many self-organizing maps (grouped in terms of time and/or spatial location) gives rise to stable results for all the calculated map sizes with good signal to noise ratio.

In one embodiment, each time based group corresponds to process state and defined by a unique identifier. In this case, the unique identifier allows for grouping the data by circumstances of the time.

In one embodiment, the spatial grouping is done by extracting engineering data of the plant, said engineering data comprising metadata containing information about logical connections of parts of the plant and information about the signals from different devices in the plant. For example, engineering tools such as COMOS PT may be provided with the possibility to extract engineering data as metadata, e.g. XML.

In one exemplary embodiment said spatial grouping comprises using the engineering data to define separating parts in the plant and to group together all parts of the plant between a pair of separating parts, wherein separating parts correspond to parts of the plant where signal or media flow starts or ends. Using separating parts for spatial grouping leads to the creation of lesser number of groups and does not incur in a loss of relationships between parts. In this embodiment, it should be noted that each separating part may belong to more than one spatial group.

In an alternate exemplary embodiment, said spatial grouping comprises using the engineering data to construct signed digraphs and, for each signal on said digraph, identifying and grouping together all related signals. Advantageously, digraphs provide coarse relationships which would simplify spatial grouping.

In accordance with a particularly advantageous embodiment, to improve the fault detection capability of the self organizing maps, the step of training said self-organizing maps comprises scaling data corresponding to one or more process tags to a desired interval.

In a further advantageous embodiment, to further improve the fault detection capability of the self organizing maps, the step of training said self-organizing maps comprises using a differential quotient for one or more process tags.

In one exemplary embodiment, the method further involves determining a cause of said deviation by deploying non-coherent fault trees that are constructed using said engineering data. Fault trees contain failure and success events, using AND, OR and NAND logic which can be used to narrow down on a possible cause of failure, i.e., deviation.

In an alternate exemplary embodiment, the method further involves determining a cause of said deviation by using signed digraphs that are constructed using said engineering data. Signed digraphs feature the advantage of including interconnections, i.e. positive and negative influences between process variables. Additionally, control loops can be considered in the model. Thus, digraphs provide increased efficiency of fault analysis and a narrower picture of possible causes.

In a contemplated embodiment, to effectively deploy the fault trees or signed digraphs, a device connected to the signal causing the largest contribution of the deviation in the self-organizing map is used as a starting point to determine the cause of the deviation.

In a further embodiment, the proposed method further comprises notifying said cause of the deviation at a maintenance station.

In another aspect of the present invention, a computer program product is provided that comprises computer readable program code embodied therein, which, when executed on a computer, carries out the method according the above described embodiments.

The present invention is further described hereinafter with reference to illustrated embodiments shown in the accompanying drawings, in which:
- FIG 1: is a schematic diagram of a process control system wherein embodiments of the present invention may be applicable,
- FIG 2: is a flowchart representing data flow for a diagnosis method in accordance with the present invention,
- FIG 3: represents a scheme of a batch process with unique identifiers (UID) for each process state,
- FIG 4: illustrates an example for a piping and instrumentation diagram which may be used for defining spatial groups,
- FIGS 5a-c: show an exemplary non-coherent fault tree in three parts, and
- FIG 6: shows a piping and instrumentation flow chart with two regions for spatial based grouping of data, in one example application of the present invention.

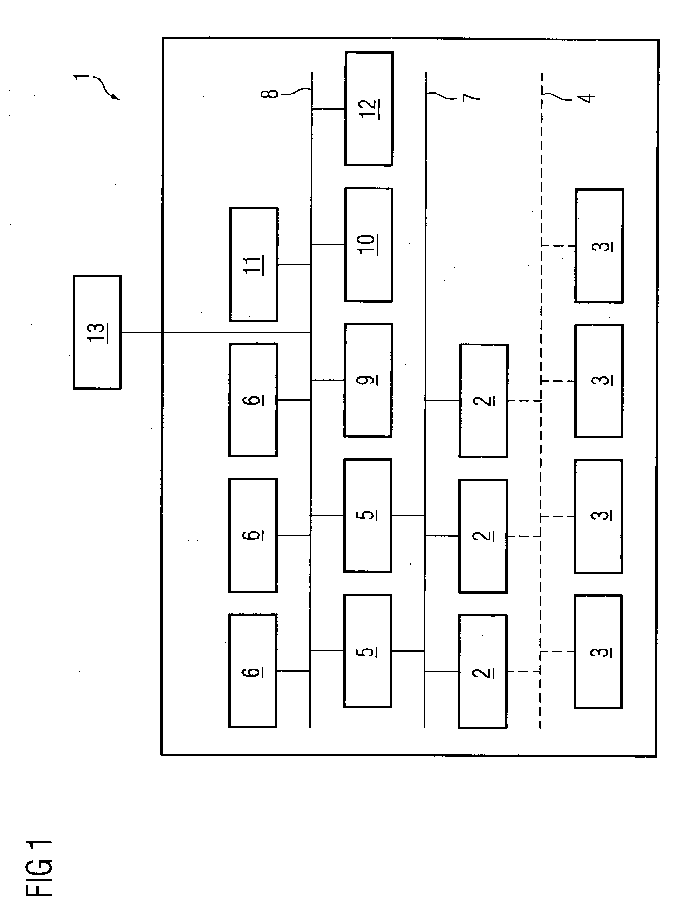

Referring now to FIG 1, a process control system 1 for controlling a process plant is illustrated, wherein the present invention may be implemented. The illustrated system 1 is a distributed control system comprising a plurality of automation systems 2, including, for example, programmable logic controllers (PLC) that are connected to field devices/actuators 3 by a field bus 4. The automation systems 2 communicate process data from these field devices/actuators 3 to one or more operating system (OS) servers 5 via communication network 7, comprising, for example, industrial Ethernet. The process data is accessed from the OS server(s) 5 by one or more OS clients 6 via terminal bus 8. A central archive server 12 may be provided for storing historical process data.

A plant status diagnosis means 13 is provided for diagnosis of plant status based on current and historical process data process data using self-organizing maps (SOM). To that end, the plant status diagnosis means 13 obtains historical process data and groups the historical process data into at least one of two ways, namely time based groups and spatial groups. Based on the grouping, a separate self-organizing map is then trained for each of the time based groups and/or spatial groups. The trained self-organizing maps are subsequently deployed by feeding the trained self-organizing maps with current process data of the plant. A fault condition is identified when a deployed self-organizing map detects a deviation in the current process data. Additionally, the plant status diagnosis means 13 may further be provided with means for determining a cause of the deviation and means for notifying a deviation and the identified cause to a maintenance station server 10, from which it is accessed by a maintenance station client 11. In the illustrated embodiment, the plant status diagnosis means 13 includes a computer system comprising appropriate software, which when executed, carries out the diagnosis method mentioned above.

The present invention may be used for both batch and continuous processes. The example illustrated in FIG 1 is a batch application, wherein the plant is controlled through different process states by a batch server 9. A batch process is a discontinuous process. Typical branches that use batch processes are biotechnology, pharmaceuticals, plastics etc. The terminology of batch processes was defined by NAMUR and ISA SP88. Batch software is used to create and modify recipes separately from the engineering system of the process control system. Recipes contain set points of the relevant process variables and instructions or procedural rules for production. Normally, several batches are processed at the same time so that some production process cells are in use and not currently available. Before the production can be started the recipes are assigned to the production cell with the required equipment and capacity. During production the process variables change in a wide range. This may lead to decreased performance and quality compared to having a well defined state. Therefore it is necessary to monitor these variables against the ideal profile.

In the context of the present discussion, the term "process state" refers to a recipe operation as a step in a batch. Additionally, for plants not featuring a batch system, the term "process state" may be referred to steps of sequential function charts (SFC).

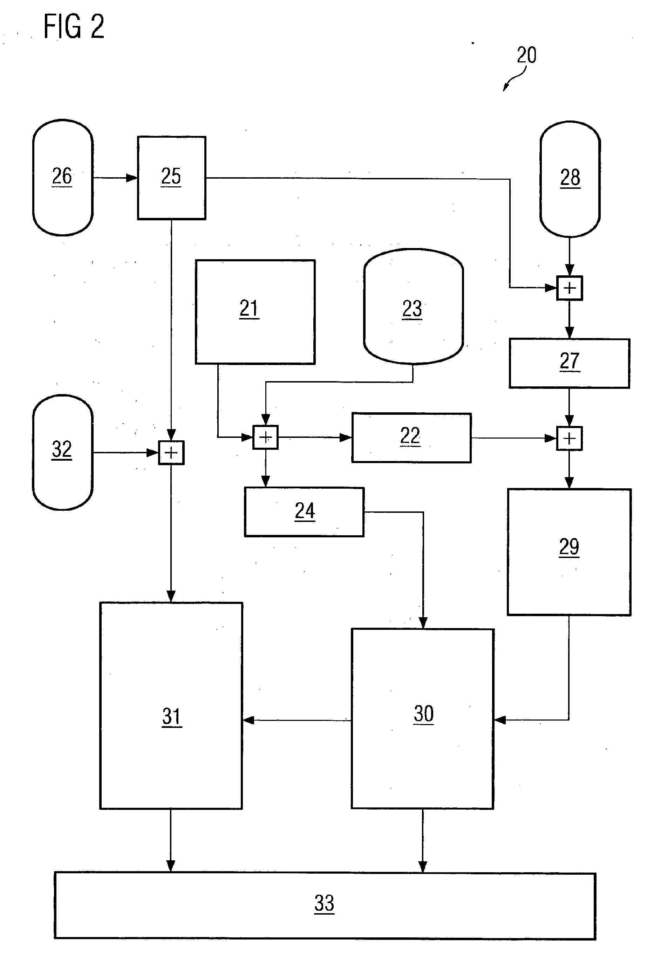

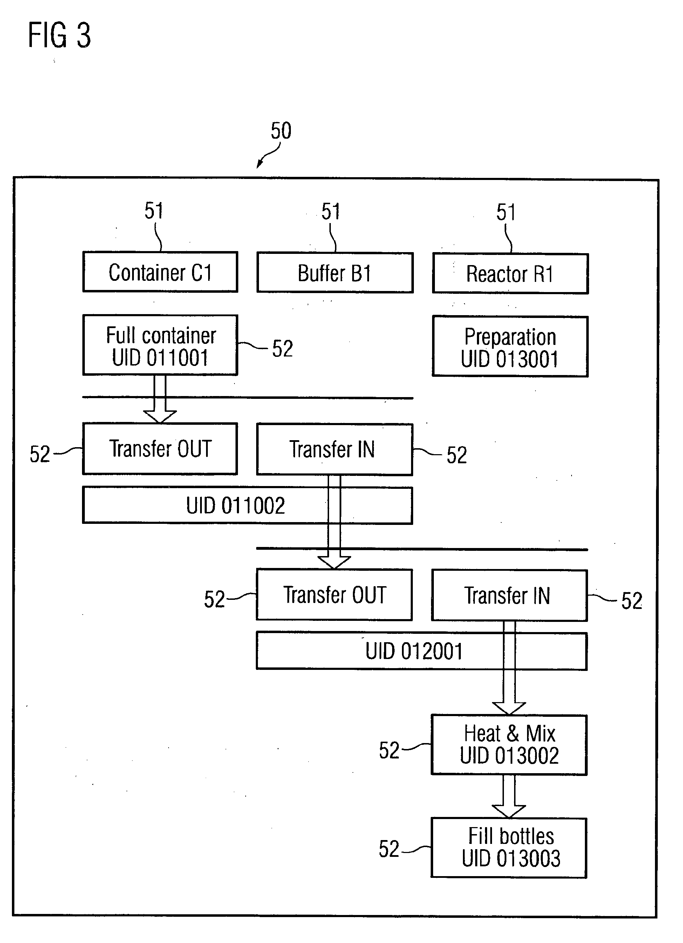

FIG 2 shows an exemplary flowchart 20 illustrating the proposed diagnosis method in greater detail. Herein, block 21 involves defining a unique identifier for each process state. For a batch process these unique identifiers correspond to recipe operations. An exemplary illustration is made referring to FIG 3, which shows a recipe procedure 50 comprising multiple sub-recipe procedures 51 and each of the sub-recipe procedures 51 comprising one or more recipe operations 52. As shown, a unique identifier (UID) is defined for each recipe operation 52.

Referring back to FIG 2, in an alternate embodiment, instead of recipe operations of a batch process, the block 21 may involve defining unique identifiers for steps in a sequential function chart (SFC). Let the unique identifiers defined in block 21 be designated as UID 1, UID 2...UID N. At block 22, historical process data is obtained from process signals 23, including, for example signals from transmitters/actuators, control signals from the distributed control system and additional signals. Using the identifiers defined at block 21, the historical process data may be grouped by circumstances of time, i.e., into time based groups. The above-obtained historical data is generally stored in a central archive server. The unique identifiers obtained at block 21 may be further stored in a short time archival system, such as an OPC server (block 24).

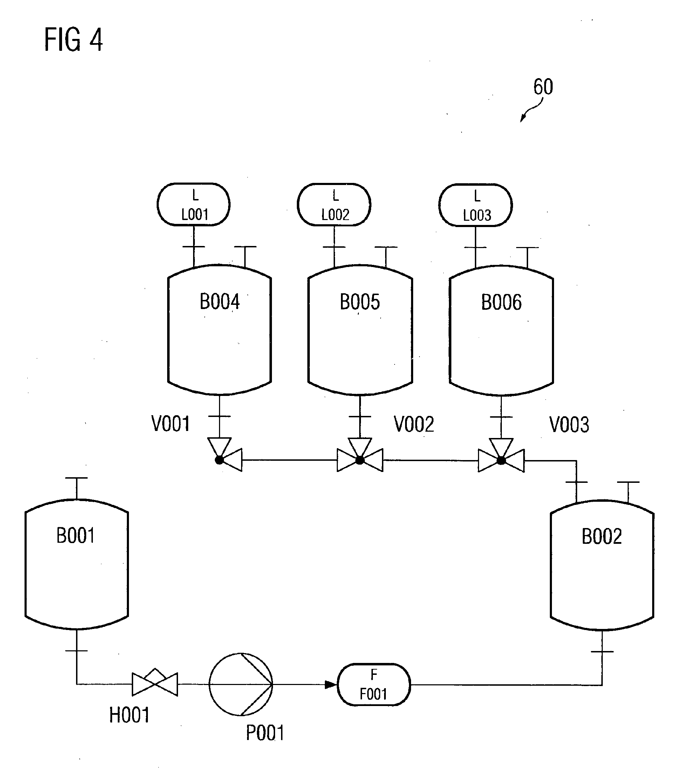

In the illustrated embodiment, process data is also grouped into spatial groups using engineering data. Accordingly, at block 25 engineering data is obtained from an engineering system 26 having an engineering tool such as COMOS PT that features the possibility to extract engineering data as metadata, e.g. XML. The metadata contains the logical connection of the parts of the plant as well as information about the signals of the different field devices. Next at block 27, using the engineering data obtained at block 25, plant components are grouped into spatial groups using one of two exemplary approaches. In a first approach, separating parts in the plant are defined. Separating parts may be all parts where flow starts or ends, e.g. all vessels. Herein, the term "flow" may refer to signal flow or media flow (for example, water, gas, etc). From the engineering metadata, all parts between separating parts are grouped together. The separating parts may belong to more than one spatial group. FIG 4 shows a simplified example of a piping and instrumentation diagram (P&ID) 60 constructed from engineering data. In this example, two spatial groups could be defined. The first group comprises the vessels B001 and B002 including the parts between said vessels. The second group comprises the vessels B002, B004, B005 and B006 including the parts between these vessels and the level transmitters L001, L002 and L003. Referring back to FIG 2, information about separating parts may be obtained using a diagnostic knowledge database 28.

A second approach for spatial grouping at block 27 includes the use of signed directed graphs (or digraphs). From the engineering data, signed digraphs can automatically be constructed which represent the interrelationships between process variables. An introduction to signed digraphs is presented in the document L.M. Bartlett, E.E. Hurdle, E.M. Kelly; Integrated System Fault Diagnostics Utilising Digraph and Fault Tree Based Approaches; Reliability Engineering and System Safety, 2009, vol.94, no.6, p.1107-15. The digraph model features interconnections or influences between variables as well as failure modes. After having constructed the digraph model, for each signal related signals are traced and grouped. The spatial groups thus formed may have a strong overlap. On the one hand, this results in coarse relationships. On the other hand, relationships not foreseen by the digraph model are lost and, additionally, lots of spatial groups may need to be created.

Referring back to FIG 2, the spatial groups of the plant components obtained at block 27 are designated herein as SG 1, SG 2, ... SG M. Using these groups, historical process data may be grouped based on spatial location of the signals. Next, at block 29, for each process state which has a unique identifier defined at block 21, and for each spatial group defined at block 27 (if the group is involved in the process state), a separate self-organizing map is trained using historical process data. Whether a group is involved in a process state can be deduced, for example from changes of operation conditions, i.e. from the change of control variables (and the groups affected by these actuators) in a process state. Using the above defined designations of the unique identifiers (UID) and spatial groups (SG), the various self-organizing maps (SOM) created at step 29 may be designated as:
SOM 11 corresponding to UID 1, SG 1,
SOM 12 corresponding to UID 1, SG 2,
SOM 1M corresponding to UID 1, SG M,...
SOM 21 corresponding to UID 2, SG 1,...
SOM NM corresponding to UID N, SG 1M.

The above-mentioned time based and spatial grouping provides several advantages. First, the map size of each self-organizing map can be smaller compared to using a single map. Searching the node with the smallest distance during operation can then be performed faster. More importantly, the number of different good states in each map is much smaller than using a single map. Thus, outliers can be identified much easier and more accurate. As described by means of an example provided below, using a small map size, for example, a map of size 2x3, is sufficient to detect various types of faults.

To further improve the fault detection capability of a self-organizing map, it is advantageous to scale the data to a desired interval, e.g. [-1;1] for all process tag types. This ensures that no process tag type is over-emphasized or underemphasized when calculating the deviation of an input vector. Further, for specific process tag types, it is advantageous to use the filtered or differential quotient (or difference quotient) in addition to or instead of the original signal. Process tag types, for which the differential quotient is useful, comprise, for example, level measurement of tanks, pressure measurement (to detect rapid pressure changes) and flow rate measurement. In these cases, the differential quotient would respectively be the rate of change of level, pressure, or flow. As the distributed control system is aware of its process tag types, the information whether to use the differential quotient can be passed and used during the training phase of the map and during evaluation as additional input channel.

Next, at block 30, the SOMs trained at block 29 are deployed during plant operation. In this step, the different self-organizing maps that are involved a process state are fed with current process data. Thus:
At process state UID=1, SOM 11 ... SOM 1M are deployed,
At process state UID=2, SOM 21 ... SOM 2M are deployed,...
At process state UID=N, SOM N1 ... SOM NM are deployed.

In the case that one of the self-organizing maps detects a deviation in the current process data at block 30, a fault or failure condition is identified. At block 31, a cause is determined for the deviation identified at block 30. Advantageously, since any map belongs to a particular spatial group, fault location is already narrowed down. In order to give possible causes, fault trees or signed digraphs may be deployed. For this, information about devices, their connection and flow direction and connected signals (such as a P&ID) can be obtained from the engineering data extracted at block 25 from engineering tools such as COMOS PT. Further, knowledge about failure states of process and P&ID parts (for fault tree analysis) and knowledge about interconnections or influences between P&ID parts (for signed digraphs) may be obtained from a diagnostic knowledge database 32.

An introduction on fault trees and signed digraphs has been presented in the above-mentioned document L.M. Bartlett, E.E. Hurdle, E.M. Kelly; Integrated System Fault Diagnostics Utilising Digraph and Fault Tree Based Approaches; Reliability Engineering and System Safety, 2009, vol.94, no.6, p.1107-15.

The use of fault trees and signed digraphs for determining a cause of a failure in accordance with the present invention is described below.

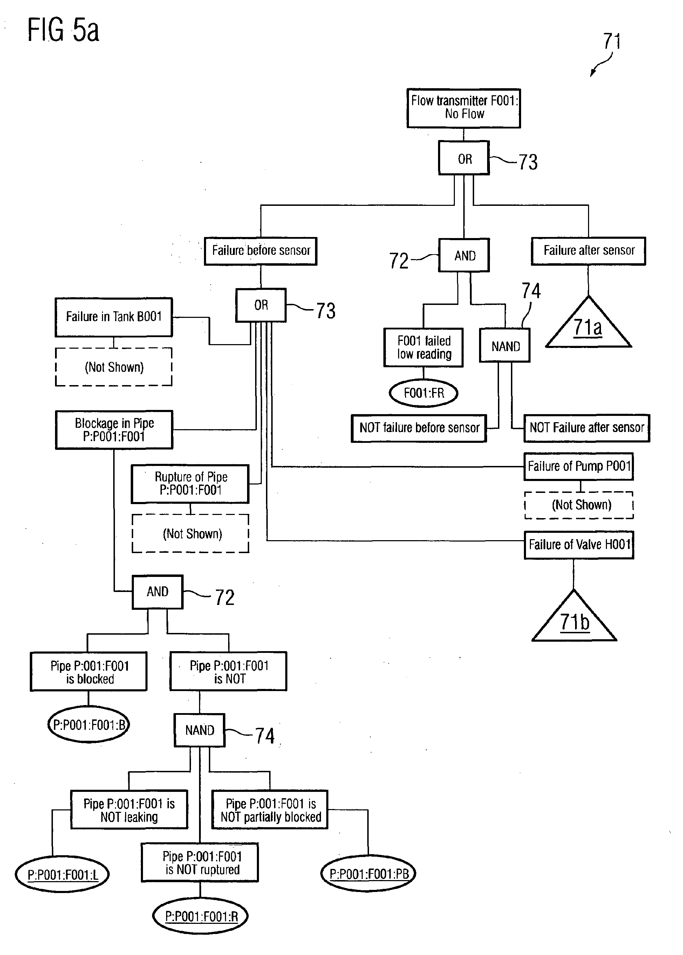

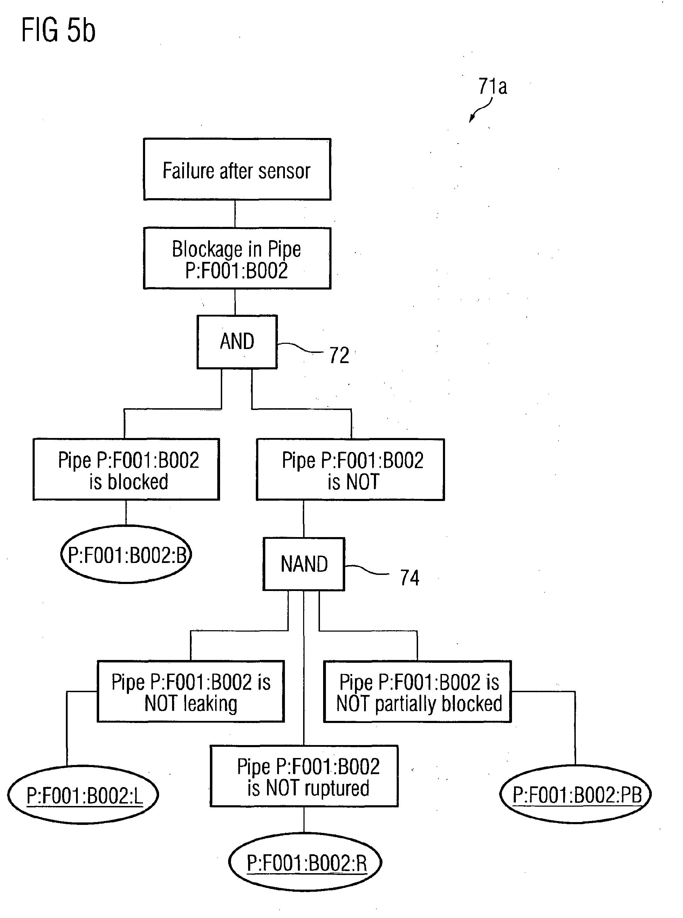

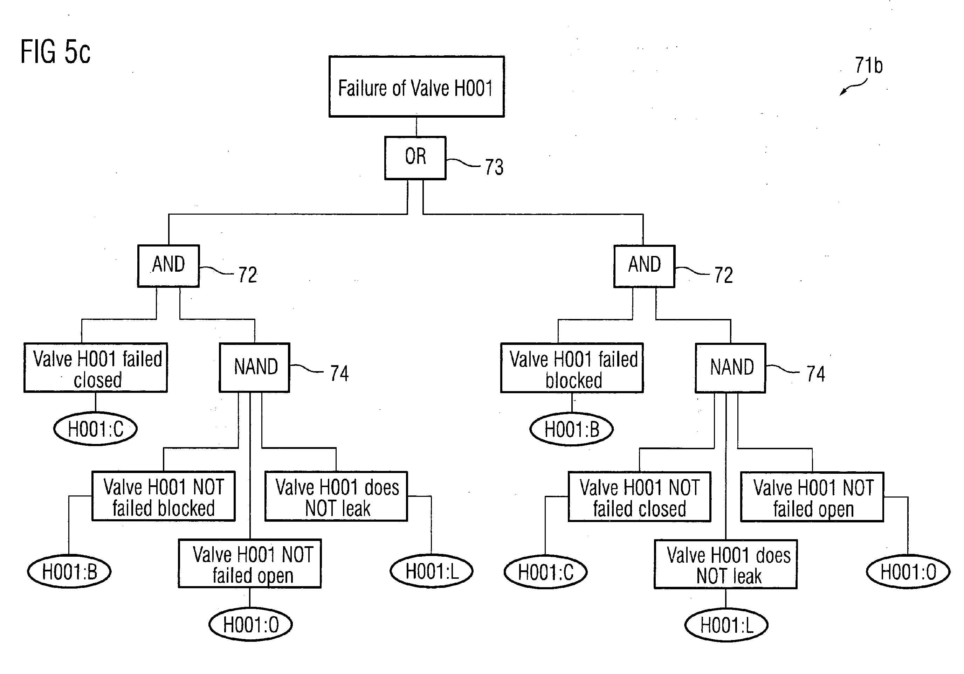

In accordance a first embodiment, non-coherent fault trees are automatically constructed using P&ID-metadata and, as diagnostic knowledge, the failure modes of plant components and logical connections between the failure modes. Fault trees contain failure and success events, therefore using AND, OR and NAND logic. FIG 5a shows an exemplary fault tree 71 constructed using engineering data and diagnostic knowledge of failure modes and FIGS 5b and 5c show the parts 71a and 71b of the main fault tree 71. As can be seen, in the fault tree, success or failure event is arrived at by a logical combination of preceding success or failure events using an AND gate 72, an OR gate 73, or a NAND gate 74. As a starting point for a fault tree, the device connected to the signal causing the largest contribution of the deviation in the self-organizing map is utilized.

In accordance with an alternate embodiment, signed digraphs are constructed for determining the cause of a fault or deviation. Again, as a starting point for signed digraph analysis, the device connected to the signal causing the largest contribution of the deviation in the self-organizing map is used. Compared to fault trees, signed digraphs feature the advantage of including interconnections, i.e. positive and negative influences between process variables. Additionally, control loops can be considered in the model. Thus, digraphs are more efficient than fault trees. Further, signed digraphs provide a narrower picture of possible causes than fault trees do.

At block 33, the identified deviations and/or the possible cause(s) of deviation is/are then displayed plant personnel at a plant maintenance station.

The advantages of the embodiments described above are summarized below. First of all, the illustrated embodiments provide enhanced real-time performance. This is because the map size of each map can be smaller compared to using a single map as a result of which searching the node with the smallest distance during operation can then be performed faster. Further outlier detection accuracy is improved as the number of different good states in each map is much smaller than using a single map, leading to a better signal-to-noise-ratio. Thus, outliers can be identified much easier and more accurate. The present invention also provides improved robustness by deploying grouping which results in fault detection for different sizes of maps. Furthermore, faults specific to a spatial group will be identified as such, speeding up fault location and diagnosis. Using engineering data combined with a distributed control system, a system for diagnosis of process plants can automatically be set up.

Several embodiments may be considered while practicing the invention. For example, for continuous processes, using time based groups can be omitted. Again, if splitting the plant into spatial groups is not possible or does not make sense, e.g. for very small plants, using spatial groups can be omitted. However, as seen in the example below, the best results are achieved when both time based and spatial grouping is carried out.

### Example

In an exemplary use case of plant diagnosis using the present invention, data was taken from a distributed control system, and the determination of fault conditions or error states was done with the help of self-organizing maps (SOM). Owing to the limited data basis, no definite statement could be made herein about the frequency of false positive (FP) alarms.

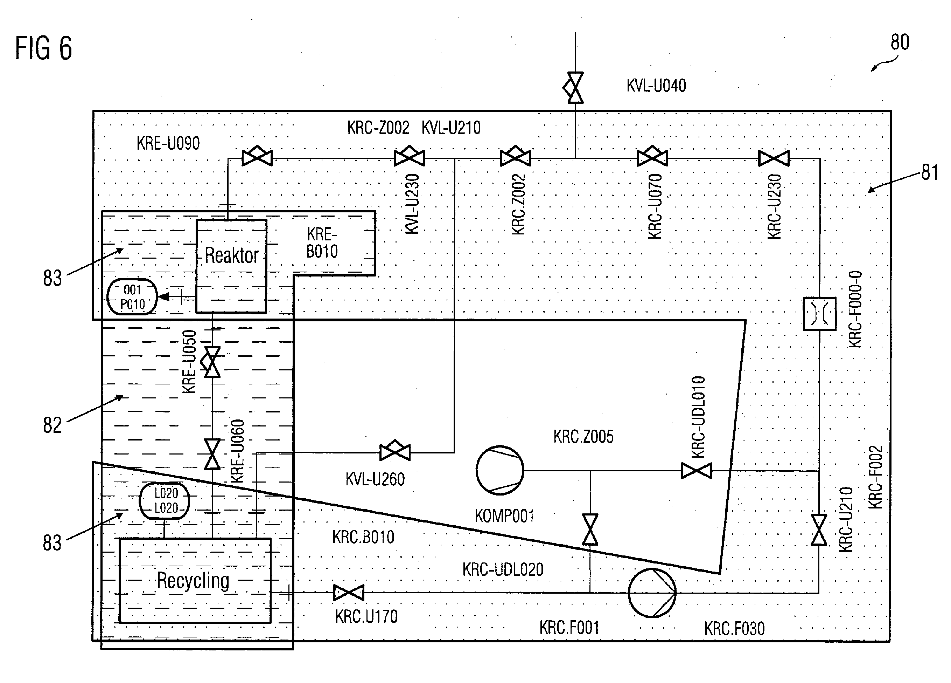

FIG 6 shows a P&ID 80 for the set up considered in this example. Herein, two spatial groups, namely SG 1 and SG 2, have been identified, which have been represented by shaded regions 81 and 82. The shaded regions 83 correspond to the overlap between the spatial groups. Table 1 below illustrates the captured signals of FIG 6 and their use.

**Table 1**

| **No** | **Name** | **binary** | **for SOM** | | **Spatial group** | | **Remark** |
|---|---|---|---|---|---|---|---|
| | | | **Used** | **Not used** | **SG 1** | **SG 2** | |
| 1 | KRC_F020 | | X | | O | | Flow through Coriolis DN50 |
| 2 | KVL_F210 | | X | | O | | Flow through MAG |
| 3 | KRC_P010 | | X | | O | * | Fill level recycling |
| 4 | KRE_P010 | | X | | O | * | Fill level reactor |
| 5 | KRE_L110 | | X | | O | * | Fill level reactor |
| 6 | Luft | | | X | | | Gas flow |
| 7 | KRC_E030 | | X | | O | | Pump |
| 8 | KRC_E030_CURRENT | | X | | O | | Pump flow |
| 9 | KRC_E030_PHI | | X | | O | | Pump cos(phi) |
| 10 | KRC_E030_RUN | X | X | | O | | Pump started |
| 11 | | | X | | O | | Pump suction pressure |
| 12 | | | X | | O | | Pump discharge pressure |
| 13 | KRE_U050 | | X | | | * | Regulating valve: reactor-> recycling |
| 14 | KRC_U070 | | X | | O | | Regulating valve: recycling -> reactor |
| 15 | KVL_U230 | | | X | | | Regulating valve (malfunction blockage) |
| 16 | KVL_P220 | | X | | O | | Pressure:recycling->reactor |
| 17 | KVL_P210 | | X | | O | | Pressure: recycling ->reactor |
| 18 | KRE_U030 | | X | | O | | Regulating valve |
| 19 | KRE_U070 | | | X | | | Discharge (malfunction external leakage (storage)) |
| 20 | KVL_U040 | X | | X | | | Discharge to the collector (malfunction external leakage) |
| 21 | KVL_U210 | | X | | O | | Regulating valve recycling -> reactor |
| 22 | KVL_U200 | X | X | | O | | Valve recycling -> reactor |
| 23 | KVL_U260 | | | X | | | Regulating valve (malfunction leakage internal) |

Table 2 below shows the malfunctions (fault conditions) identified for the use case. Herein, sccm means standard cubic centimeters per minute and SID refers to malfunction identification number.

**Table 2**

| **No.** | **SID** | **Malfunction name** | **Explanation** | **implementation** |
|---|---|---|---|---|
| 1 | 1 | Normal | No malfunction | Armature KRC-U170 is closed |
| 2 | 2 | Dry run | No inflow before the pump | |
| 3 | 3 | Blockage | Blockage after the pump | Regulating valve KVL-U230 is closed (0%) |
| 4 | 4 | Partial blockage | Partial blockage after the pump | Regulating valve KVL-U230 is set to 10% |
| 5 | 6 | External leakage | Outflow in recycling container | KV1-U040 is opened |
| 6 | 7 | Internal leakage (small) | Backflow from recycling container directly to recycling container | KV1-U260 is opened slightly |
| 7 | 8 | Internal leakage (big) | Backflow from recycling container directly to recycling container | KV1-U260 is opened more |
| 8 | 9 | External leakage (storage) | Outflow from reactor to storage | KRE-U070 is opened |
| 9 | 1005 | Air Coriolis (500 sccm) | Air injection before Coriolis measurement device (500 sccm) | |
| 10 | 1010 | Air Coriolis (1000 sccm) | Air injection before Coriolis measurement device (1000 sccm) | |
| 11 | 1050 | Air Coriolis (5000 sccm) | Air injection before Coriolis measurement device (5000 sccm) | |
| 12 | 1200 | Air Coriolis (20000 sccm) | Air injection before Coriolis measurement device (20000 sccm) | |
| 13 | 2005 | Air pump (500 sccm) | Air injection before pump (500 sccm) | |
| 14 | 2020 | Air pump (2000 sccm) | Air , injection before pump (20000 sccm) | |

For determining process states, a sequential function chart (SFC) was created and a unique identifier (UID) was assigned to each step of the process using the SFC. The process states are listed in Table 3 below.

**Table 3**

| **No.** | **UID** | **Name** | **Evaluated** | **Comment** |
|---|---|---|---|---|
| 1 | 0 | No UID | | Invalid UID |
| 2 | 1 | Starting | | Start phase 1 |
| 3 | 2 | Starting 2 | | Start phase 2 |
| 4 | 10 | Starting 3 | | Start phase 3 |
| 5 | 20 | Pumps 100% | X | Pumps from recycling to reactor, regulating valve opened 100% |
| 6 | 21 | Pumps 90% | X | Pumps from recycling to reactor, regulating valve opened 90% |
| 7 | 22 | Pumps 80% | X | Pumps from recycling to reactor, regulating valve opened 80% |
| 8 | 23 | Pumps 70% | X | Pumps from recycling to reactor, regulating valve opened 70% |
| 9 | 30 | Intermediate 1 | | Turn off pump, close valves |
| 10 | 31 | Intermediate 2 | | Open valves |
| 11 | 40 | Let out rector | X | Back flow from reactor to recycling container |
| 12 | 50 | End 1 | | Closing of valves |
| 13 | 51 | End 2 | | SFC ended |

The data captured in Table 1 was read into various SOMs. The evaluation was done in four ways, namely, a) using a single SOM, b) using multiple SOMs having data grouped by time (i.e., using time based grouping only), c) using multiple SOMs having data grouped by location (i.e., using spatial grouping only), and d) using multiple SOMs having data grouped by time and location (i.e., using both time based grouping and spatial grouping)

The process of evaluation was implemented in MATLAB and based on an exemplary procedure similar to that disclosed in the document WO 03063017 A2**.** The time based grouping was done with the help of the UIDs (please refer to Table 3), while the spatial grouping was done based on the spatial groups SG 1 and SG 2, represented by the shaded regions 81 and 82 in FIG 6. As was noted, the two spatial groups have an overlap, as indicated by the region 83 in the same figure.

Map sizes from at least 2x3 to 30x40 were computed. The training was done with 670 records. The test data consisted of 10576 records on the whole.

The deviation (fault condition) was computed as follows:
1. A reference map was computed from the training data (maximum value of each row).
2. A threshold value was defined (which was 0.2 in this case).
3. The test data was computed (test map).
4. The reference map was deducted from the test map.
5. The threshold value was deducted.
6. All the values that are greater than zero were added up (sum).
7. The sum was
   a. Divided by the number of cells in the map (deviation weighted by number of cells)
   b. Divided by the number of cells in the map whose value is greater than zero (deviation weighted by number of affected cells)
8. False negative (FN) alarms were obtained, if, for the data of the given malfunction, no cell of the map(s) has exceeded the threshold value.

### Results

On the whole, for each of the four evaluation methods (single map, time based, spatial based and spatial and time based), 15 different map sizes were computed. Table 4 shows the number of map sizes for which at least one malfunction was not recognized.

**Table 4**

| **On the whole, 15 computed map sizes** | **Number of map sizes for which at least one malfunction was not recognized** | **Maximum number of malfunctions that were not recognized** |
|---|---|---|
| Single map | 6 | 5 |
| Time based | 0 | - |
| Spatial based | 7 | 5 |
| Spatial and time based | 0 | - |

The computation methods "single map" and "spatial based", for which one or two maps were computed, show false negative alarms for six and seven map sizes respectively. The evaluation methods "time based" (5 maps) and "Spatial and time based" (10 maps), on the other hand, recognized all the malfunctions correctly for all map sizes. This means that grouping into several maps will result in a significantly more robust result. The best results in each case for the four categories of maps are shown in Table 5 below.

**Table 5**

| **Data based on 15 computed map sizes** | **Best results (without false negative alarms)** | | | | | | |
|---|---|---|---|---|---|---|---|
| | **Deviation weighted by number of cells** | | | | **Deviation weighted by number of affected cells** | | |
| | **mean** | **min** | **max** | **map size** | **mean** | **min** | **max** |
| single map | 0.12 | 0.01 | 0.53 | 8x8 | 0.74 | 0.27 | 1.70 |
| | 0.07 | 0.005 | 0.36 | 10x15 | 0.81 | 0.24 | 1.97 |
| time based | 0.31 | 0.05 | 1.85 | 2x3 | 0.72 | 0.31 | 2.22 |
| | 0.04 | 0.01 | 0.14 | 20x30 | 1.27 | 0.34 | 2.71 |
| spatial based | 0.12 | 0.01 | 0.56 | 8x8 | 0.65 | 0.28 | 1.45 |
| | 0.05 | 0.004 | 0.22 | 15x20 | 0.69 | 0.25 | 1.32 |
| spatial and | 0.34 | 0.05 | 2.00 | 2x3 | 0.74 | 0.31 | 2.40 |
| time based | 0.04 | 0.01 | 0.14 | 20x30 | 1.27 | 0.33 | 2.72 |

Here, it is seen that the mean value and the minimum value for "time based" and "spatial and time based" is significantly higher than that for the "single map" and "spatial based" methods. The use of many maps thus gives a significantly better "signal to noise ratio" than if one (or two) map(s) are used. Furthermore, it may be seen from Table 5 that if many maps are used, even the smallest map size (2x3) gives these stable results. Small map sizes can be computed faster with small memory requirements than larger maps.

While this invention has been described in detail with reference to certain preferred embodiments, it should be appreciated that the present invention is not limited to those precise embodiments. Rather, in view of the present disclosure which describes the current best mode for practicing the invention, many modifications and variations would present themselves, to those of skilled in the art without departing from the scope and spirit of this invention. The scope of the invention is, therefore, indicated by the following claims rather than by the foregoing description. All changes, modifications, and variations coming within the meaning and range of equivalency of the claims are to be considered within their scope.

## Claims

1. A method (20) for diagnosis of status of a process plant, comprising:
- obtaining (22) historical process data of said plant,
- grouping (21, 27) said historical process data into a plurality of time based groups and a plurality of spatial groups, wherein the spatial grouping (27) is done by extracting (25) engineering data of the plant, the engineering data comprising metadata containing information about logical connections of parts of the plant and information about the signals from different devices in the plant, and wherein said spatial grouping (27) comprises using that engineering data to define separating parts in the plant and to group together all parts of the plant between a pair of separating parts, wherein separating parts correspond to parts of the plant where signal flow or media flow starts or ends,
- training (29) a plurality of self-organizing maps using said historical process data, wherein each self-organizing map corresponds to a combination of one of said time based groups and one of said spatial groups,
- deploying (30) the trained self-organizing maps under plant operation, comprising feeding the trained self-organizing maps with current process data of said plant, and
- determining a fault condition when a deployed self-organizing map detects a deviation in the current process data.

2. The method (20) according to claim 1, wherein each time based group corresponds to process state (53) and defined by a unique identifier.

3. The method (20) according to claim 1 or 2, wherein said spatial grouping (27) comprises using the engineering data to construct signed digraphs and, for each signal on said digraph, identifying and grouping together all related signals.

4. The method (20) according to any of the preceding claims, wherein the step of training (29) said self-organizing maps comprises scaling data corresponding to one or more process tags to a desired interval.

5. The method (20) according to any of the preceding claims, wherein the step of training (29) said self-organizing maps comprises using a differential quotient for one or more process tags.

6. The method (20) according to any of the preceding claims, further comprising determining (31) a cause of said deviation by deploying non-coherent fault trees that are constructed using said engineering data.

7. The method (20) according to any of claims 1 to 5, further comprising determining (31) a cause of said deviation by using signed digraphs that are constructed using said engineering data.

8. The method (20) according to any of claims 6 and 7, wherein a device connected to the signal causing the largest contribution of the deviation in the self-organizing map is used as a starting point to determine the cause of the deviation.

9. The method (20) according to any of claims 6 to 8, further comprising notifying (33) said cause of the deviation at a maintenance station.

10. A computer program product comprising computer readable program code embodied therein, which, when executed on a computer, carries out the method according to any of claims 1 to 9.

11. A process control system (1) having plant status diagnosis means (13), the plant diagnosis means further comprising:
- means for obtaining (22) historical process data of said plant,
- means for grouping (21, 27) said historical process data into a plurality of time based groups and a plurality of spatial groups, wherein the spatial grouping (27) is done by extracting (25) engineering data of the plant, the engineering data comprising metadata containing information about logical connections of parts of the plant and information about the signals from different devices in the plant, and wherein said spatial grouping (27) comprises using that engineering data to define separating parts in the plant and to group together all parts of the plant between a pair of separating parts, wherein separating parts correspond to parts of the plant where signal flow or media flow starts or ends,
- means for training (29) a plurality of self-organizing maps using said historical process data, wherein each self-organizing map corresponds to a combination of one of said time based groups and one of said spatial groups,
- means for deploying (30) the trained self-organizing maps under plant operation, comprising feeding the trained self-organizing maps with current process data of said plant, and
- means for determining a fault condition when a deployed self-organizing map detects a deviation in the current process data.

12. The system (1) according to claim 11, wherein each time based group corresponds to process state (53), further wherein the system (1) comprises means for providing a unique identifier to each process state (53).

13. The system (1) according to any of claims 11 and 12, further comprising means for determining (31) a cause of said deviation by deploying non-coherent fault trees or signed digraphs that are constructed using said engineering data.

14. The system (1) according to claim 13, further comprising means for notifying (33) said cause of the deviation at a maintenance station.

## Patentansprüche

1. Verfahren (20) zur Zustandsdiagnose einer Prozessanlage, Folgendes umfassend:
- Erhalten (22) historischer Prozessdaten der Anlage,
- Gruppieren (21, 27) der historischen Prozessdaten in mehrere zeitbasierte Gruppen und mehrere räumliche Gruppen, wobei das räumliche Gruppieren (27) durch Extrahieren (25) von technischen Daten der Anlage vorgenommen wird, wobei die technischen Daten Metadaten umfassen, welche Informationen über logische Verknüpfungen von Anlagenteilen und Informationen über die Signale von verschiedenen Vorrichtungen in der Anlage enthalten, und wobei das räumliche Gruppieren (27) ein Verwenden dieser technischen Daten umfasst, um trennende Teile in der Anlage zu definieren und alle Anlagenteile zwischen einem Paar trennender Teile zusammen zu gruppieren, wobei trennende Teile Anlagenteilen entsprechen, bei welchen ein Signalfluss oder Medienfluss beginnt oder endet,
- Trainieren (29) mehrerer selbstorganisierender Abbildungen unter Verwendung der historischen Prozessdaten, wobei jede selbstorganisierende Abbildung einer Kombination aus einer der zeitbasierten Gruppen und einer der räumlichen Gruppen entspricht,
- Einsetzen (30) der trainierten selbstorganisierenden Abbildungen unter Anlagenbetrieb, umfassend Speisen der trainierten selbstorganisierenden Abbildungen mit aktuellen Prozessdaten der Anlage, und
- Bestimmen eines Fehlerzustands, wenn eine eingesetzte selbstorganisierende Abbildung eine Abweichung in den aktuellen Prozessdaten detektiert.

2. Verfahren (20) nach Anspruch 1, wobei jede zeitbasierte Gruppe einem Prozesszustand (53) entspricht und durch eine eindeutige Kennung definiert ist.

3. Verfahren (20) nach Anspruch 1 oder 2, wobei das räumliche Gruppieren (27) ein Verwenden der technischen Daten, um mit Vorzeichen versehene Digrafen zu konstruieren, und für jedes Signal auf dem Digrafen ein Identifizieren und Zusammengruppieren aller verwandten Signale umfasst.

4. Verfahren (20) nach einem der vorhergehenden Ansprüche, wobei der Schritt des Trainierens (29) der selbstorganisierenden Abbildungen ein Skalieren von Daten entsprechend einer oder mehrerer Prozessmarken zu einem gewünschten Intervall umfasst.

5. Verfahren (20) nach einem der vorhergehenden Ansprüche, wobei der Schritt des Trainierens (29) der selbstorganisierenden Abbildungen ein Verwenden eines Differentialquotienten für eine oder mehrere Prozessmarken umfasst.

6. Verfahren (20) nach einem der vorhergehenden Ansprüche, weiterhin umfassend ein Bestimmen (31) einer Ursache der Abweichung durch Einsetzen nicht kohärenter Fehlerbäume, welche unter Verwendung der technischen Daten aufgebaut werden.

7. Verfahren (20) nach einem der Ansprüche 1 bis 5, weiterhin umfassend ein Bestimmen (31) einer Ursache der Abweichung durch Verwenden mit Vorzeichen versehener Digrafen, welche unter Verwendung der technischen Daten aufgebaut werden.

8. Verfahren (20) nach einem der Ansprüche 6 und 7, wobei eine Vorrichtung, welche mit dem Signal verknüpft ist, welches den größten Beitrag zu der Abweichung in der selbstorganisierenden Abbildung bewirkt, als ein Anfangspunkt verwendet wird, um die Ursache der Abweichung zu bestimmen.

9. Verfahren (20) nach einem der Ansprüche 6 bis 8, weiterhin umfassend ein Benachrichtigen (33) einer Wartungsstation über die Ursache der Abweichung.

10. Computerprogrammprodukt, welches einen computerlesbaren Programmcode umfasst, welcher darin enthalten ist, welcher, wenn er auf einem Computer ausgeführt wird, das Verfahren nach einem der Ansprüche 1 bis 9 ausführt.

11. Prozesssteuerungssystem (1) mit einem Diagnosemittel (13) eines Anlagenzustands, wobei das Anlagendiagnosemittel weiterhin Folgendes umfassend:
- Mittel zum Erhalten (22) historischer Prozessdaten der Anlage,
- Mittel zum Gruppieren (21, 27) der historischen Prozessdaten in mehrere zeitbasierte Gruppen und mehrere räumliche Gruppen, wobei das räumliche Gruppieren (27) durch Extrahieren (25) von technischen Daten der Anlage vorgenommen wird, wobei die technischen Daten Metadaten umfassen, welche Informationen über logische Verknüpfungen von Anlagenteilen und Informationen über die Signale von verschiedenen Vorrichtungen in der Anlage enthalten, und wobei das räumliche Gruppieren (27) ein Verwenden dieser technischen Daten umfasst, um trennende Teile in der Anlage zu definieren und alle Anlagenteile zwischen einem Paar trennender Teile zusammen zu gruppieren, wobei trennende Teile Anlagenteilen entsprechen, bei welchen ein Signalfluss oder Medienfluss beginnt oder endet,
- Mittel zum Trainieren (29) mehrerer selbstorganisierender Abbildungen unter Verwendung der historischen Prozessdaten, wobei jede selbstorganisierende Abbildung einer Kombination aus einer der zeitbasierten Gruppen und einer der räumlichen Gruppen entspricht,
- Mittel zum Einsetzen (30) der trainierten selbstorganisierenden Abbildungen unter Anlagenbetrieb, umfassend Speisen der trainierten selbstorganisierenden Abbildungen mit aktuellen Prozessdaten der Anlage, und
- Mittel zum Bestimmen eines Fehlerzustands, wenn eine eingesetzte selbstorganisierende Abbildung eine Abweichung in den aktuellen Prozessdaten detektiert.

12. System (1) nach Anspruch 11, wobei jede zeitbasierte Gruppe einem Prozesszustand (53) entspricht, wobei weiterhin das System (1) Mittel zur Bereitstellung einer eindeutigen Kennung für jeden Prozesszustand (53) umfasst.

13. System (1) nach einem der Ansprüche 11 und 12, weiterhin umfassend Mittel zum Bestimmen (31) einer Ursache der Abweichung durch Einsetzen nicht kohärenter Fehlerbäume oder mit Vorzeichen versehener Digrafen, welche unter Verwendung der technischen Daten aufgebaut werden.

14. System (1) nach Anspruch 13, weiterhin umfassend ein Mittel zum Benachrichtigen (33) einer Wartungsstation über die Ursache der Abweichung.

## Revendications

1. Procédé ( 20 ) de diagnostic d'un état d'une installation dans lequel s'effectue des processus, comprenant :
- obtenir ( 22 ) des données historiques de processus de l'installation,
- regrouper ( 21 , 27 ) ces données historiques de processus en une pluralité de groupes temporels et en une pluralité de groupes spatiaux, le regroupement ( 27 ) spatial étant effectué en extrayant ( 25 ) des données d'ingénierie de l'installation, les données d'ingénierie comprenant des métadonnées contenant de l'information sur des connexions logiques de parties de l'installation et de l'information sur les signaux provenant de dispositifs différents de l'installation, et le regroupement ( 27 ) spatial comprenant l'utilisation de ces données d'ingénierie pour définir des parties de séparation dans l'installation et pour regrouper ensemble toutes les parties de l'installation entre une paire de parties de séparation, les parties de séparation correspondant à des parties de l'installation ou des flux de signaux ou des flux de fluides commencent ou se terminent,
- faire subir un apprentissage ( 29 ) à une pluralité de cartes s'organisant d'elles-mêmes en utilisant les données historiques de processus, chaque carte s'organisant de soi-même correspondant à une combinaison de l'un de ces groupes temporels et de l'un de ces groupes spatiaux,
- déployer ( 30 ) les cartes ayant subi un apprentissage et s'organisant d'elles-mêmes, sous un fonctionnement de l'installation comprenant renseigner les cartes ayant subi un apprentissage et s'organisant d'elles-mêmes par des données présentes de processus de l'installation et
- déterminer un état de défaut, lorsqu'une carte déployée et s'organisant de soi-même détecte un écart dans les données présentes de processus.

2. Procédé ( 20 ) suivant la revendication 1, dans lequel chaque groupe temporel correspond à un état ( 53 ) de processus et est défini par un identificateur unique.

3. Procédé ( 20 ) suivant la revendication 1 ou 2, dans lequel le regroupement ( 27 ) spatial comprend utiliser les données d'ingénierie pour construire des digraphes signés et, pour chaque signal sur le digraphe, identifier et regrouper ensemble tous les signaux en relation.

4. Procédé ( 20 ) suivant l'une quelconque des revendications précédentes, dans lequel le stade d'apprentissage ( 29 ) des cartes s'organisant d'elles-mêmes comprend mettre à l'échelle les données correspondant à une ou à plusieurs étiquettes de processus à un intervalle souhaité.

5. Procédé ( 20 ) suivant l'une quelconque des revendications précédentes, dans lequel le stade d'apprentissage ( 29 ) des cartes s'organisant d'elles-mêmes comprend utiliser un quotient différentiel pour une ou pour plusieurs étiquettes de processus.

6. Procédé ( 20 ) suivant l'une quelconque des revendications précédentes, comprenant en outre déterminer ( 31 ) une cause de l'écart en déployant des arbres de défaut non cohérents, qui sont construits en utilisant les données d'ingénierie.

7. Procédé ( 20 ) suivant l'une quelconque des revendications 1 à 5, comprenant en outre déterminer ( 31 ) une cause de l'écart en utilisant des digraphes signés, qui sont construits en utilisant les données d'ingénierie.

8. Procédé ( 20 ) suivant l'une quelconque des revendications 6 et 7, dans lequel un dispositif connecté au signal contribuant le plus à l'écart dans la carte s'organisant de soi-même est utilisé comme point de départ pour déterminer la cause de l'écart.

9. Procédé ( 20 ) suivant l'une quelconque des revendications 6 à 8, comprenant en outre notifier ( 33 ) la cause de l'écart à un poste d'entretien.

10. Produit de programme d'ordinateur comprenant un code de programme pouvant être exécuté par un ordinateur qui y est incorporé et qui, lorsqu'il est exécuté sur un ordinateur, effectue le procédé suivant l'une quelconque des revendications 1 à 9.

11. Système ( 1 ) de commande de processus ayant des moyens ( 13 ) de diagnostic d'un état d'une installation, les moyens de diagnostic de l'installation comprenant en outre :
- des moyens d'obtention ( 22 ) de données historiques de processus de l'installation,
- des moyens de regroupement ( 21 , 27 ) des données historiques de processus en une pluralité de groupes temporels et en une pluralité de groupes spatiaux, le regroupement ( 27 ) spatial étant effectué en extrayant ( 25 ) des données d'ingénierie de l'installation, les données d'ingénierie comprenant des métadonnées contenant de l'information sur des connexions logiques de parties de l'installation et de l'information sur les signaux provenant de dispositifs différents de l'installation, et le regroupement ( 27 ) spatial comprenant l'utilisation de ces données d'ingénierie pour définir des parties de séparation dans l'installation et pour regrouper ensemble toutes les parties de l'installation entre une paire de parties de séparation, les parties de séparation correspondant à des parties de l'installation où des flux de signaux ou des flux de fluides commencent ou se terminent,
- des moyens pour faire subir un apprentissage ( 29 ) à une pluralité de cartes s'organisant d'elles-mêmes en utilisant les données historiques de processus, chaque carte s'organisant de soi-même correspondant à une combinaison de l'un des groupes temporels et de l'un des groupes spatiaux,
- des moyens pour déployer ( 30 ) les cartes ayant subi un apprentissage et s'organisant de soi-même sous un fonctionnement de l'installation, comprenant renseigner les cartes ayant subi l'apprentissage et s'organisant de soi-même par des données présentes de processus de l'installation et
- des moyens de détermination d'un état de défaut, lorsqu'une carte déployée s'organisant de soi-même détecte un écart dans les données présentes de processus.

12. Système ( 1 ) suivant la revendication 11, dans lequel chaque groupe temporel correspondant à un état ( 53 ) de processus, dans lequel, en outre, le système ( 1 ) comprend des moyens pour fournir un identificateur unique à chaque état ( 53 ) de processus.

13. Système ( 1 ) suivant l'une quelconque des revendications 11 et 12, comprenant en outre des moyens de détermination ( 31 ) d'une cause de l'écart en déployant des arbres de défaut non cohérents ou des digraphes signés, qui sont construits en utilisant les données d'ingénierie.

14. Système ( 1 ) suivant la revendication 13, comprenant en outre des moyens de notification ( 33 ) de la cause de l'écart à un poste d'entretien.
